(19) Europäisches Patentamt
European Patent Office
Office européen des brevets

(11) Veröffentlichungsnummer: **0 012 246 B1**

(12) **EUROPÄISCHE PATENTSCHRIFT**

(45) Veröffentlichungstag der Patentschrift: **20.05.81**

(21) Anmeldenummer: **79104611.3**

(22) Anmeldetag: **20.11.79**

(51) Int. Cl.³: **C 12 P 1/02,** A 23 L 1/23, A 24 B 15/30, C 11 B 9/00 // C12P7/02, C12P7/26, C12P17/04, C07D307/79, C07C35/18, C07C49/173

(54) **Riech- und Geschmackstoffgemische, deren fermentative Herstellung, deren Verwendung und Produkte, die sie enthalten.**

(30) Priorität **24.11.78 CH 12045/78**

(43) Veröffentlichungstag der Anmeldung: **25.06.80 Patentblatt 80/13**

(45) Bekanntmachung des Hinweises auf die Patenterteilung: **20.05.81 Patentblatt 81/20**

(84) Benannte Vertragsstaaten: **CH DE FR GB NL**

(56) Entgegenhaltungen:
**— Keine —**

(73) Patentinhaber: **L. Givaudan & Cie Société Anonyme, Patentdienst Postfach, CH-4002 Basel (CH)**

(72) Erfinder: **Krasnobajew, Victor, Dr., Zürichstrasse 125, CH-8700 Küsnacht (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

# Riech- und Geschmackstoffgemische, deren fermentative Herstellung, deren Verwendung und Produkte, die sie enthalten

Die vorliegende Erfindung betrifft ein Verfahren zur Herstellung von Riech- und/oder Geschmackstoffgemischen durch Fermentierung von Jononkörpern mit Mikroorganismen der Genera Botryodiplodia, Botryosphaeria oder Lasiodiplodia.

Als Ausgangsmaterialien für das erfindungsgemäße Verfahren verwendet man Verbindungen der allgemeinen Formel

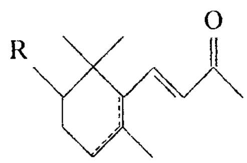

(I)

worin R Wasserstoff oder Methyl und eine der gestrichelten Linien eine zusätzliche Bindung darstellen.

Erfindungsgemäß können alle Stämme der Genera Botryodiplodia, Botryosphaeria und Lasiodiplodia sowie Varietäten davon, verwendet werden. Bevorzugte Stämme sind insbesondere:

| | |
|---|---|
| Botryodiplodia theobromae | IFO-6469 |
| Botryosphaeria rhodina | CBS-356.59 |
| Botryosphaeria rhodina | CBS-175.26 |
| Botryosphaeria rhodina | CBS-306.58 |
| Lasiodiplodia theobromae | ATCC-10936 und |
| Lasiodiplodia theobromae | ATCC-28570 |

Diese Stämme zeichnen sich insbesondere durch ausgezeichnete Transformations-Kapazität (Biokonversion, siehe Tabelle II, Kolonne 3) aus.

Die Mikroorganismen können als Kultur(-suspension) (worunter das Mycel und die entsprechende Nährlösung verstanden wird), in Form des Mycels oder in aufbereiteter Form, z. B. in Form eines aus der Kultursuspension oder dem Mycel in an sich bekannter Weise hergestellten Enzymextrakts verwendet werden. Die Kultursuspension kann durch Beimpfen eines geeigneten Mediums mit dem Mikroorganismus hergestellt werden. Geeignete Medien sind solche, die Kohlenstoffquellen, Stickstoffquellen, anorganische Salze u. a. für das Wachstum des Mikroorganismus geeignete Nährstoffe enthalten. Al Kohlenstoffquelle kommen beispielsweise Glukose, Fructose, Saccharose, Melasse, Dextrin, Maltose, Mannose, Stärke, z. B. Maisstärke, Lactose und Glycerin in Betracht; Stickstoffquellen sind beispielsweise stickstoffhaltige, organische Substanzen wie Pepton, Fleischextrakt, Hefeextrakt, Sojamehl, Maisquellwasser (Cornsteep liquor) und Kasein, Harnstoff, Aminosäuren, oder stickstoffhaltige, anorganische Verbindungen wie Nitrate und anorganische Ammoniumsalze. Als anorganische Salze sind Phosphate bzw. Natrium-, Kalium-, Magnesium-, Calcium-, Mangan-, Eisen- und Kupfersalze zu erwähnen.

Die Züchtung des Mikroorganismus kann als Submerskultur, als Schüttelkultur oder als stationäre Kultur (z. B. in Fermentern) durchgeführt werden; vorzugsweise wird der Mikroorganismus unter aeroben Bedingungen kultiviert.

Zweckmäßig arbeitet man in einem pH-Bereich von 3–8, insbesondere zwischen 4–7,5. Wenn nötig, können zur pH-Regulation anorganische oder organische Säuren wie Salzsäure, Essigsäure, Oxalsäure, oder Basen, wie z. B. NaOH, oder Puffersysteme wie Phosphat-, Phthalat-, Tris-(hydroxymethyl)-aminomethan-Systeme, oder Zucker, wie z. B. Glucose eingesetzt werden.

Die Reaktionstemperatur liegt allgemein zwischen 15 und 35° C, insbesondere zwischen 24–32° C.

Das erfindungsgemäße Verfahren wird zweckmäßig so durchgeführt, daß die Verbindung der allgemeinen Formel I als Substrat zu der angezüchteten, sich im Wachstum befindlichen Kulturlösung gegeben wird. Die Konzentration des Substrates beträgt zweckmäßig 0,5 g pro Liter bis 20 g pro Liter. Die erfindungsgemäße Fermentation kann durch Fortsetzung der Kultivierung des Mikroorganismus unter den obenerwähnten Bedingungen durchgeführt werden. Die Reaktionszeit kann in Abhängigkeit von Spezies und Stamm des verwendeten Mikroorganismus, von der Zusammensetzung des Kulturmediums, vom verwendeten Substrat und dessen Konzentration variieren. Im allgemeinen genügt eine Fermentationszeit von 18–216 Stunden, insbesondere 18–144 Stunden. Die Reaktionstemperatur liegt allgemein zwischen 15 und 35° C, insbesondere zwischen 24–32° C.

Die erfindungsgemäße Fermentation kann auch mit dem aus der Kulturlösung isolierten Mycel des Mikroorganismus oder mit einem aus der Kulturlösung oder dem Mycel in an sich bekannter Weise hergestellten Enzymextrakt durchgeführt werden. In diesem Falle kann die Fermentation zweckmäßig in wäßriger Lösung, z. B. einer Pufferlösung, in physiologischer Salzlösung, in frischer Nährlösung oder in Wasser durchgeführt werden.

Das Substrat I kann als solches oder als Lösung in einem hydrophilen Lösungsmittel, wie Aceton, Dimethylsulfoxid, Methanol, Äthanol, Äthylenglykol, Propylenglykol oder Dioxan zugesetzt werden. Man kann auch einer wäßrigen Suspension des Substrats ein oberflächenaktives Mittel oder ein Dispersionsmittel zusetzen, oder das Substrat durch Behandlung mit Ultraschall emulgieren.

Zwecks Schaumbekämpfung können konventionelle Anti-Schaummittel verwendet werden, also z. B. Siliconöle (z. B. UCON), Polyalkylenglykol-Derivate, oder Mais- oder Sojabohnenöl.

Das Substrat kann der Kultur des Mikroorganismus während der Anzüchtung oder aber, wie oben erwähnt, nach beendeter Züchtung zugesetzt werden.

Der Zeitpunkt der Zugabe der Verbindung I zum vorgezüchteten Mikroorganismus kann von Bedeutung sein. Einerseits ist es wünschenswert, ein möglichst dichtes Wachstum der Mikroorganismen zu erzielen (größere Biomasse), andererseits kann eine Überalterung der Kultur (die sich beispielsweise in Sporulation und Farbveränderung äußern kann) bei zu langer Züchtungsdauer auf die Transformation von I einen negativen Einfluß haben. Bei vielen Mikroorganismen fällt der pH-Wert anfangs um eine halbe bis 2 Einheiten (bedingt durch die Produktion von Säure). Wenn der pH-Wert wieder zu steigen beginnt (Lyse!) und die Biomasse der Kultur (schwach oder kaum) noch zunimmt, ist dies in der Regel der günstigste Zeitpunkt, um die Verbindung I zuzusetzen. Es ist dies der Zeitpunkt des Abschlusses des exponentiellen Wachstums der Biomasse (Bestimmung: Zentrifugieren und Wägen). Der optimale Zeitpunkt der Zugabe der Verbindung I kann auch — wenn in Anwesenheit von Glucose gearbeitet wird — mittels Glucoseanalysator bestimmt werden (Verbrauch der zugegebenen Glucose). Änderungen des pH-Werts während der Vorkultur können jedoch auch auftreten in Abhängigkeit von dem verwendeten Mikroorganismus und dem verwendeten Medium.

Der Verlauf der Transformation von I kann mit Hilfe bekannter gaschromatographischer oder dünnschichtchromatographischer Methoden verfolgt werden. Wird demzufolge eine Starke Abnahme oder sogar gänzliches Verschwinden von I festgestellt, so wird zweckmäßigerweise das Ausgangsmaterial wieder zugegeben, um eine möglichst hohe Ausnützung der Transformationskapazität des Mikroorganismus zu erreichen.

Das Fermentationsprodukt kann aus dem Reaktionsgemisch (der Fermentationsbrühe) in an sich bekannter Weise isoliert werden, beispielsweise durch Extraktion (welche Methode bevorzugt ist) mit einem organischen Lösungsmittel, das mit Wasser nicht mischbar ist, wie Chloroform, Methylenchlorid (bevorzugt) oder Methylacetat, Tetrachlorkohlenstoff, Butanol, Diäthyläther, Essigester (bevorzugt). Bei der Extraktion mit einem organischen Lösungsmittel können Emulsionen auftreten. Diese können unter Verwendung der dem Fachmann geläufigen Methoden aufgebrochen werden, z. B. durch Verwendung von Filterhilfen, wie Cellit, Zentrifugieren usw.

Das organische Extraktionsmittel, gegebenenfalls nach Trocknung des Extraktes mit Natriumsulfat, kann durch Destillation entfernt werden; das Transformationsprodukt bleibt hierauf als gelbliches bis bräunliches Öl zurück. Zwecks Entfärbung kann nun eine Behandlung mit Aktivkohle erfolgen. Das Reaktionsgemisch kann aber auch einer schonenden Destillation unter Hochvakuum, z. B. bei Drucken von 0,01 bis 0,5 Torr unterzogen werden. Eine Auftrennung des Transformationsproduktes in einzelne Komponenten kann durch Chromatographie (z. B. Dickschicht, wie Säulenchromatographie) auf Trägern wie Aluminiumoxyd, Silicagel oder Cellulose erfolgen.

Es ist klar, daß auch die Mutanten der erwähnten Genera und der spezifisch genannten Mikroorganismen dieselbe Transformation von I zeitigen. Solche Mutanten können auf an sich bekannte Art und Weise leicht durch Einwirkung von UV-Strahlen, Röntgenstrahlen oder übliche mutagene Substanzen, wie z. B. Acridinorange, auf die entsprechenden Sporen oder Mycelsuspensionen erhalten werden.

Die erfindungsgemäß erhältlichen, isolierten Gemische stellen gelblich bis bräunlich gefärbte Flüssigkeiten dar, sind unlöslich in Wasser, aber löslich in organischen Lösungsmitteln, wie z. B. Alkoholen, Äthern, Ketonen, Estern, Kohlenwasserstoffen und halogenierten Kohlenwasserstoffen.

Zur Charakterisierung bzw. Identifizierung der nach dem erfindungsgemäßen Verfahren erhältlichen Gemische dienen gewisse in diesen Gemischen enthaltene Verbindungen, die oft daraus isoliert, zumindest aber darin analytisch (beispielsweise durch Gaschromatographie, gekoppelt mit Massenspektroskopie) nachgewiesen werden können. Es sind dies die folgenden Verbindungen:

| Ausgangs-material | Gemisch enthält (1) | | (2) |
|---|---|---|---|
| α-Ionon | 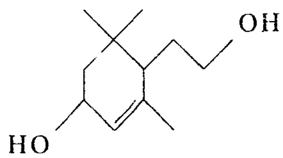 (1) | und/oder | 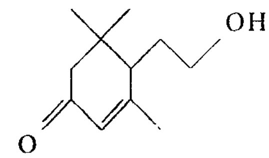 (2) |
| β-Ionon | 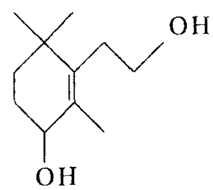 (5) | und/oder | 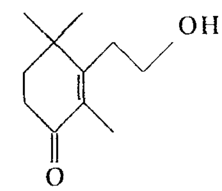 (6) |
| α-Iron | 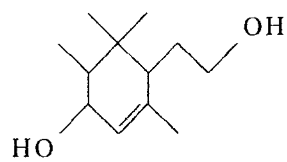 (7) | und/oder | 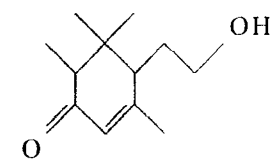 (8) |
| β-Iron | 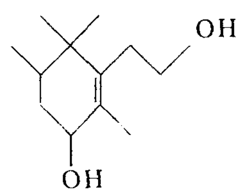 (11) | und/oder | 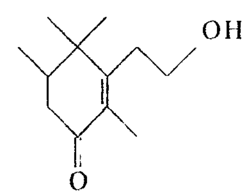 (12) |

(1) dehydratisiert und cyclisiert spontan zu

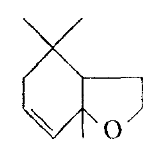

(3)

(2) cyclisiert spontan zu

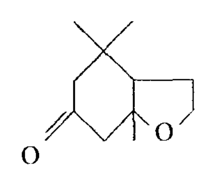

(4)

(7) dehydratisiert und cyclisiert spontan zu

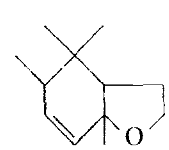

(9)

(8) cyclisiert spontan zu

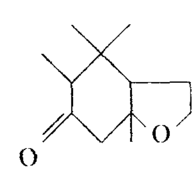

(10)

Die Identifizierung kann so geschehen, daß das Fermentationsprodukt aus der Fermentationsbrühe, wie oben beschrieben, durch Extraktion mit einem organischen Lösungsmittel z. B. Essigester (3×, Volumen = 1 : 1) isoliert wird. Der Extrakt kann hierauf über $Na_2SO_4$ getrocknet und das Lösungsmittel abdestilliert werden. Hierauf kann in möglichst wenig, z. B. 10 ml Äther zu Hexan = 1 : 1 eine Lösung erstellt werden, und die Lösung mittels Säulenchromatographie an Kieselgel, beispielsweise der 30fachen Menge, z. B. Silicagel Merck, unter Verwendung von Äther-zu-Hexan-Gemischen mit steigendem Anteil an Äther in Fraktionen aufgetrennt werden, wobei zweckmäßigerweise mit 30 : 3 (Hexan zu Äther) begonnen wird, und mit Äther allein aufgehört wird. Die Eluate können mittels konventioneller analytischer Methoden untersucht werden (IR, NMR, MS).

MS:

Zur spektroskopischen Charakterisierung wurden Proben von Riechstoffgemischen verwendet, welche durch Chromatographie an der 30fachen Menge Kieselgel (Merck, Kieselgel 60; Korngröße 0,063–0,2 mm, 70–230 Mesh) aufgetrennt wurden.

Daten für die Verbindungen

(1) 184 ($M^+$, 2); 169 (15); 151 (13); 133 (14); 123 (28); 107 (23); 97 (55); 84 (100); 69 (65); 55 (34); 41 (77).

(2) 167 ($M-CH_3$, 2); 164 (3); 149 (5); 138 (16); 126 (41); 109 (7); 95 (100); 83 (9); 67 (30); 55 (8); 41 (46).

(3) 166 ($M^+$, 9); 151 (100); 107 (16); 83 (25); 55 (23); 43 (77).

(4) 182 ($M^+$, 1); 167 (8); 125 (100); 83 (56); 55 (35); 43 (90).

(5) 184 ($M^+$, 24); 169 (12); 139 (100); 128 (70); 121 (23); 109 (30); 81 (18); 72 (22); 55 (15); 43 (40):

(6) 182 ($M^+$, 2); 167 (78); 149 (18); 137 (62); 126 (80); 109 (95); 93 (55); 81 (62); 67 (68); 55 (80); 43 (100).

(10) 196 ($M^+$, 1); 181 (51); ; 139 (10); 125 (100); 111 (17); 97 (14); 83 (44); 55 (34); 43 (30).

(11) 198 ($M^+$, 15); 183 (5); 153 (80); 135 (22); 128 (48); 107 (38); 95 (54); 83 (47); 72 (70); 55 (38); 43 (80).

(12) 196 (M, 9); 181 (100); 163 (7); 151 (32); 139 (20); 126 (63); 111 (35); 97 (19); 81 (16); 69 (11); 55 (13); 41 (15).

Die Gemische weisen besondere organoleptische Eigenschaften auf, auf Grund derer sie sich vorzüglich als Riech- und/oder Geschmackstoffe eignen.

Die Erfindung betrifft demgemäß auch die Verwendung dieser Gemische als Riech- und/oder Geschmackstoffe.

Die erfindungsgemäß als Riech- und/oder Geschmackstoffe verwendeten Gemische können demgemäß beispielsweise zur Parfümierung bzw. Aromatisierung von Produkten, wie Kosmetika (Seifen, Salben, Pudern, Zahnpasten, Mundwässern, Desodorantien, Shampoos, Lotionen, Eaux de Toilette, Eaux de Cologne, Extraits usw.), Waschmitteln, Detergentien, Raucherartikeln, bzw. Nahrungsmitteln, Genußmitteln und Getränken dienen, wobei die Verbindungen vorzugsweise nicht allein, sondern in Form von Kompositionen mit anderen Riech- bzw. Geschmackstoffen eingesetzt werden. Solche Riech- bzw. Geschmackstoffkompositionen mit einem Gehalt an den erfindungsgemäß erhältlichen Gemischen bilden ebenfalls Gegenstand der Erfindung. Ihre Herstellung geschieht auf an sich bekannte Art: Zugabe der Gemische zu bekannten Riech- bzw. Geschmackstoffkompositionen oder Vermischung mit als Bestandteil von Riech- bzw. Geschmackstoffkompositionen geeigneten natürlichen oder synthetischen Verbindungen oder Gemischen.

Als Riech- und/oder Aromastoffe eignen sich die Riechstoff- und/oder Geschmackstoffgemische aufgrund ihrer wertvollen olfaktischen Eigenschaften, insbesondere in Kombination mit einer umfangreichen Reihe von natürlichen und synthetischen Riechstoffen bzw. Aromastoffen wie z. B.

— Galbanumöl Mastixöl, Vetiveröl, Patchouliöl, Patchouliblätteröl, Sandelholzöl, Cedernöl, Fichtenöl, Baummoos absolue, Basilikumöl, Beifußöl, Kamillenöl, Wurmsamenöl, Selleriesamenöl, Angelikasamenöl, Thymianöl, Rosmarinöl, Lavendelöl, Lavandinöl, Aspiköl, Salbeiöl, Petitgrainöl, Neroliöl, Bergamotteöl, Citronenöl, Mandarinenöl, Orangenöl, Grapefruitöl, Geraniumöl, Benzoe-resinoid, Melilotus absolue, Jasmin absolue, Rosenöl, Ylang-Ylang-Öl, Corianderöl, Veilchenblätter absolue, Tuberosen absolue usw.,

- mit Aldehyden wie Hydroxycitronellal, Zyklamenaldehyd, alpha-Hexylzimtaldehyd, 3,5-Dimethyl-cyclohex-3-en-1-yl-carboxaldehyd, Citral, Citronellal, 2,6-Dimethyl-6-hepten-1-al, Methylnonylacetaldehyd, Undecanal, Anisaldehyd usw.,

- mit Ketonen wie alpha-Jonon, beta-Jonon, Acetanisol, 4-(para-Hydroxyphenyl)-2-butanon, Campher, Menthon, Carvon, Pulegon usw.,

- mit Acetalen und Ketalen wie Phenylacetaldehyd-dimethylacetal, Phenylacetaldehyd-glycerinacetal, 2-Methyl-1,3-dioxolan-2-äthylacetat, Capronaldehyd-dimethylacetal usw.,

- mit Äthern wie Eugenolmethyläther, Methyl-1-methyl-cyclododecyläther, Anethol, Estragol usw.,

- mit phenolischen Körpern wie Eugenol, Isoeugenol, Kresol usw.,

- mit Alkoholen wie cis-3-Hexanol, trans-2-cis-6-Nonadienol, cis-6-Noneol, Linalool, Geraniol, Nerol, Citronellol, Nerolidol, Benzylalkohol, Phenyläthylalkohol usw.,

- mit Estern wie Methyldihydrojasmonat, Linalylacetat, Geranylacetat, Cedrylacetat, Vetiverylacetat, para-tert.-Butylcyclohexylacetat, ortho-tert.-Butylcyclohexylacetat, Benzylacetat, Benzylsalicylat, Styrallylacetat, Äthyl-$\alpha$-methylphenylglycidat usw.,

- mit Lactonen wie $\gamma$-Undecalacton, $\gamma$-Decalacton, $\gamma$-Nonalacton, $\delta$-Decalacton, $\delta$-Octalacton, Cumarin usw.,

- mit Säuren wie Milchsäure, Buttersäure, $\alpha$-Methylbuttersäure, trans-2-Hexensäure, trans-2-Octensäure usw.,

- mit moschus- und ambraartig riechenden Körpern wie Äthylenbrassylat, 4-Acetyl-6-tert.-butyl-1,1-dimethyl-indan, 12-Oxahexadecanolid, 8$\alpha$,12-Oxido-13,14,15,16-tetranorlabdan usw.,

- mit schwefelhaltigen Verbindungen wie p-Menthan-8-thiol-3-on, Dimethylsulfid und anderen Sulfiden und Disulfiden usw.,

- mit stickstoffhaltigen Verbindungen wie Methylanthranilat, Indol, Isobutylchinolin, verschiedenen Pyrazinen usw.

Neben diesen originellen Effekten in Riechstoffkompositionen, beispielsweise vom Typ Chypre, Cologne, Tabak, Holz bzw. allgemein blumiger Richtung, ist es aber auch möglich, mit den erfindungsgemäßen Verbindungen neuartige Parfümerie-Komplexe herzustellen, nämlich die üblichen klassischen Typen in Richtung leichterer und spritzigerer Noten zu modifizieren.

Die Konzentration der erfindungsgemäß erhältlichen Gemische — die sich insbesondere auch durch ihre ausgeprägte Haftfestigkeit auszeichnen — kann je nach dem Verwendungszweck innerhalb weiter Grenzen variieren, beispielsweise zwischen etwa 0,01 (Detergentien) und etwa 15 Gew.-% (alkoholische Lösungen). In Parfümbasen bzw. Konzentraten können die Konzentrationen selbstverständlich auch höher liegen. Die Parfümbasen können in üblicher Weise zur Parfümierung von Eaux de Cologne, Eaux de Toilette, Lotionen, Cremes, Shampoos, Seifen und Detergentien usw., verwendet werden.

Als Geschmackstoffe können die Gemische beispielsweise zur Erzeugung bzw. Verbesserung, Verstärkung, Steigerung oder Modifizierung von Frucht- oder Beerenaromen in Nahrungsmitteln (Joghurt, Süßwaren usw.); in Genußmitteln (Tee usw.), Getränken (Limonaden usw.) und Tabak verwendet werden.

Die ausgeprägten geschmacklichen Qualitäten der Gemische ermöglichen die Verwendung in geringen Konzentrationen. Eine geeignete Dosierung umfaßt beispielsweise den Bereich von 0,1—100 ppm, vorzugsweise von 1—20 ppm im Fertigprodukt, d. h. dem aromatisierten Nahrungsmittel, Genußmittel oder Getränk.

Bei der Aromatisierung von beispielsweise Tabak kann die Dosierung jedoch auch höher liegen und einen größeren Bereich bestreichen, beispielsweise den Bereich von 1 bis 1000 ppm, vorzugsweise 5 bis 500 ppm.

Tabelle

In der folgenden Tabelle sind einige Effekte zusammengestellt, wie sie sich mit den erfindungsgemäßen Gemischen in verschiedenen Aromen erzielen lassen.

| Gemisch | Aroma | Dosierung | Effekt |
|---|---|---|---|
| B (Beispiel 2) | Himbeere | ppm im Fertigprodukt 0,1–30 ppm insb. 0,5–4 ppm | größere Natürlichkeit des Fruchtcharakters, volles Aroma der reifen Früchte |
| B (Beispiel 5) | Aprikose | ppm im Fertigprodukt 0,1–50 ppm insb. 0,2–5 ppm | ausgeprägte Fruchtigkeit, natürlicheres Aroma (sowohl geruchlich als auch geschmacklich) |
| B (Beispiele 2, 5) | Tabak | ppm im Tabak 1–1000 ppm insb. 5–500 ppm | beim Verrauchen abgerundetere Tabaknote, daneben Verstärkung des Tabakgeschmackes |

Die Gemische können auf übliche Weise mit den für Geschmackstoffkompositionen verwendeten Bestandteilen vermischt bzw. solchen Aromen zugesetzt werden. Unter den erfindungsgemäß verwendeten Aromen werden Geschmackstoffkompositionen verstanden, die sich auf an sich bekannte Art verdünnen bzw. in eßbaren Materialien verteilen lassen. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Für die Herstellung solcher üblicher Gebrauchsformen kommen beispielsweise folgende Trägermaterialien, Verdickungsmittel, Geschmackstoffverbesserer, Gewürze und Hilfsingredientien usw. in Frage:

Gummi arabikum, Tragant, Salze oder Brauereihefe, Alginate, Carrageen oder ähnliche Absorbentien; Maltol, Gewürzoleoresine, Raucharomen; Gewürznelken, Natriumcitrat; Mononatriumglutamat, Dinatrium-inosin-5'-monophosphat (IMP), Dinatriumguanosin-5-phosphat (GMP); oder spezielle Aromastoffe, Wasser, Äthanol, Propylenglykol, Glycerin.

## Beispiele

Tabelle 1

A. Zusammensetzung geeigneter Wachstums-Medien

| | | |
|---|---|---|
| Medium 1 | Glucose | 50,0 g |
| | Cornsteep liquor (Maisquellwasser) | 10,0 g |
| | KCl | 0,5 g |
| | $KH_2PO_4$ | 1,0 g |
| | $NaNO_3$ | 1,0 g |
| | Dest. Wasser ad | 1000 ml, |
| | pH = 6,3 vor Sterilisation | |
| | (121° im Autoklaven) | |
| Medium 2 | Glucose | 20,0 g |
| | Hefeextrakt | 3,0 g |
| | $NaNO_3$ | 1,0 g |
| | Glycin | 1,0 g |
| | $KH_2PO_4$ | 1,0 g |
| | $MgSO_4 \cdot 7\ H_2O$ | 0,5 g |
| | KCl | 0,5 g |
| | $FeSO_4 \cdot 7\ H_2O$ | 0,01 g |
| | Dest. Wasser ad | 1000 ml, |
| | pH = 6,3 vor Sterilisation | |
| Medium 3 | Glucose | 10,0 g |
| | Distillers solubles (Vinasse der Alkoholdestillation) | 10,0 g |
| | NaCl | 5,0 g |
| | $NaNO_3$ | 1,0 g |
| | $CaCO_3$ | 2,5 g |
| | Leitungswasser ad | 1000 ml, |
| | pH = 8,0 nach Sterilisation | |
| Medium 4 | Cornsteep liquor | 1,0 g |
| | $NH_4H_2PO_4$ | 3,0 g |
| | $CaCO_3$ | 2,5 g |
| | Sojaöl | 2,2 g |
| | Hefeextrakt | 2,5 g |
| | Glucose | 10,0 g |
| | Dest. Wasser ad | 1000 ml, |
| | pH = 7,6 vor Sterilisation | |
| Medium 5 | Glucose | 10,0 g |
| | Cornsteep liquor | 10,0 g |
| | Leitungswasser ad | 1000 ml, |
| | pH = 7,2 vor Sterilisation | |
| Medium 6 | Malzextrakt | 20,0 g |
| | Soja-Mehl | 15,0 g |
| | Caseinhydrolysat | 1,0 g |
| | Hefeextrakt | 1,0 g |
| | NaCl | 1,0 g |
| | Dest. Wasser ad | 1000 ml, |
| | pH = 6,3 vor Sterilisation | |

Tabelle II

B. Verwendete Mikroorganismen (Spezies) und Wachstumsmedien

| Fungus | Kulturen-Sammlung Nr. | Bio-transformation: optimal eingesetzte Mengen I in g/l | Wachstums-Medien (siehe Tabelle I) |
|---|---|---|---|
| Botryodiplodia theobromae | IFO-6469 | 1–6 | 1, 2, 3, 5, 6 |
| Botryodiplodia plamarum | CBS-142.52 | 0,2–2 | 1, 6 |
| Botryosphaeria Species rhodina | CBS-356.59 | 0,5–6 | 1, 2, 3, 6 |
| desgl. | CBS-175.26 | 1–4 | 1, 2, 3, 6 |
| desgl. | CBS-306.58 | 1–8 | 1, 2, 3, 4, 5, 6 |
| desgl. | CBS-230.30 | 0,5–2 | 1, 6 |
| desgl. | CBS-110.11 | 0,5–3 | 1, 2, 6 |
| desgl. | CBS-374.54 | 0,5–4 | 3, 6 |
| desgl. | CBS-124.13 | 0,5–4 | 1, 2, 4, 6 |
| Lasiodiplodia theobromae | ATCC-9055 | 1–3 | 1, 2, 4, 6 |
| desgl. | ATCC-10936 | 1–10 | 1, 2, 6 |
| desgl. | ATCC-16391 | 0,5–2 | 1, 2, 6 |
| desgl | ATCC-28570 | 1–8 | 1, 2, 4, 5, 6 |

## Beispiel 1

### Anzüchtung des Mikroorganismus

Die Mikroorganismen der Tabelle II werden auf Agar-Platten (hergestellt mit Medium 2) angezüchtet. Zu diesem Zweck werden mit Hilfe einer Platinöse Sporen oder Hyphae des jeweiligen Mikroorganismus auf die Oberfläche der Agarplatten ausgestrichen und 8–10 Tage bei 28° C bebrütet. Das Inokulum für die Vorkulturen wird hergestellt, indem man Sporen dieser Platten in 9 ml einer physiologischen NaCl-Lösung suspendiert und 1 ml der Sporensuspension zu 100 ml des entsprechenden Wachstum-Mediums in einem 500-ml-Schüttelkolben (Erlenmeyerkolben mit Schikanen) zugibt. Der Kolben wird bei 28° C 48 Stunden auf einen Rotationsschüttler (150–200 rpm) inkubiert. 8 ml der resultierenden Mycelsuspension werden verwendet, um Vorkulturen von 400 ml in 1-l-Schüttelkolben zu inokulieren. Nach einer Inkubationszeit von 24–48 Stunden unter den obigen Bedingungen resultiert eine dichte Mycel-Kultur (100–200 g/l nasses Mycel, wenn bei 8000 g abzentrifugiert).

### Riechstoffkomposition A, ausgehend von $\alpha$-Jonon

Ein 10-l-Glasfermenter (Biologische Verfahrenstechnik, Basel), gefüllt mit 8 l des Mediums 1 und 1 ml Antischaummittel UCON LB 625, wird mit 400 ml einer Vorkultur von Botryodiplodia theobromae IFO-6469, 24 Stunden auf demselben Medium gewachsen, angeimpft. Die Fermentationsbedingungen sind die folgenden: Rührsystem: Umwälzrührer (400 rpm), Temperatur: 28°C, Belüftung: 5 l Luft/Minute. Nach einer Fermentationszeit von 24 Stunden ist ein dichtes Wachstum der Mycel-Kultur erreicht (150 g abzentrifugiertes feuchtes Mycel/l [Biomasse]). Der pH der Kultur sinkt dabei von 6,3 auf 5,4. Zu diesem Zeitpunkt werden 2 g $\alpha$-Jonon zur Kultur gegeben und der pH-Wert wird mittels 1 n-NaOH auf pH 6,5 eingestellt und während der fortlaufenden Fermentation mit Hilfe eines automatischen Titrators auf diesem Wert gehalten. Nach 16stündiger Fermentation zeigt die gaschromatographische Analyse einer entnommenen Probe das Verschwinden von $\alpha$-Jonon an.

Hierauf werden 28 g α-Jonon innert 185 Stunden mittels einer Dosierpumpe in dem Maße zugegeben, daß stets ca. 5% α-Jonon, bezogen auf die gebildeten Produkte, vorliegen (gaschromatographische Bestimmung). Nach der angegebenen Zeit hat sich die Transformationskapazität des Mikroorganismus weitgehend erschöpft. Nun wird der Fermenterinhalt mittels 2 n-HCl auf einen pH-Wert von 2–3 eingestellt und die Fermentationsbrühe mit Methylenchlorid (dreimaliges Ausschütteln mit je 4 l) extrahiert. Die vereinigten Extrakte werden über $Na_2SO_4$ getrocknet, filtriert und das Methylenchlorid abdestilliert. Es werden 35 g eines hellbräunlichen Öls mit einem angenehmen Geruch nach Tabak und Honig erhalten. Zur Entfärbung wird das Öl in 200 ml $CH_2Cl_2$ mit 10 g Tierkohle behandelt, filtriert und das $CH_2Cl_2$ entfernt. Die Riechstoffkomposition A eignet sich vorzüglich als Komponente für Tee- und Tabaknoten. Die Riechstoffkomposition wird durch Anwesenheit der Verbindung 1 und 2, vor allem aber 4, charakterisiert.

### Beispiel 2

### Riechstoffkomposition B, ausgehend von β-Jonon

Ein 10-l-Glasfermenter (siehe Beispiel 1), gefüllt mit 8 l des Mediums 6 und 2 ml Antischaummittel (Polypropylenglykol 2000), wird mit 400 ml einer 24 Stunden alten Vorkultur von Botryodiplodia theobromae IFO 6469, gewachsen auf demselben Medium, angeimpft. Die Bedingungen der nun folgenden Fermentation sind die des Beispiels 1. Nach 24 Stunden ist dichtes Wachstum der Mycel-Kultur erreicht (180 g/l). Der pH-Wert der Kulturbrühe sinkt dabei von 6,3 auf 5,2 ab. Nun werden 5 g β-Jonon zum Fermenterinhalt gegeben, dessen pH-Wert mittels 1 n-NaOH auf pH 6,5 eingestellt und durch automatische Titration auf diesem Wert gehalten wird. Gleichzeitig wird eine Pumpe angeschlossen, die innert 5 Tagen kontinuierlich weitere 30 g β-Jonon zur Fermentationsbrühe zuführt. Die Umsetzung des β-Jonons wird gaschromatographisch verfolgt. Nach Ablauf der angegebenen Zeit wird die Zugabe des β-Jonons eingestellt und noch weitere 16 Stunden fermentiert. Zu diesem Zeitpunkt sind nur noch Spuren von β-Jonon vorhanden. Die Aufarbeitung der Fermentationsbrühe geschieht analog zu Beispiel 1. Es werden 40,2 g eines bräunlichgefärbten Öls mit angenehmem Geruch nach Tabak erhalten. Das auf diese Weise erhaltene Öl eignet sich vorzüglich als Komponente für Kompositionen sowohl männlicher wie weiblicher Linien, z. B. für Chypre oder Tabak; in letzteren Noten wird insbesondere Abrundung erzielt. Das Öl ist ferner für Himbeeraromen verwendbar. Die Riechstoffkomposition wird durch Anwesenheit der Verbindung 5 charakterisiert.

### Beispiel 3

### Riechstoffkomposition B, ausgehend von β-Jonon

Die Herstellung der Riechstoffkomposition erfolgt in Analogie zu Beispiel 2. Jedoch wird anstelle von B. theobromae IFO 6469 der Mikroorganismus Botryosphaeria rhodina CBS 306.58 verwendet. Es werden 42 g β-Jonon umgesetzt und 40 g eines bräunlichgefärbten Öles mit angenehmem Tabakgeruch erhalten. Dieses Transformationsprodukt von β-Jonon eignet sich vorzüglich als Riechstoffkomponente in Parfümkompositionen (siehe Beispiel 2). Die Riechstoffkomposition wird durch Anwesenheit der Verbindung 5 und 6 charakterisiert.

### Beispiel 4

### Riechstoffkomposition B, ausgehend von β-Jonon

Ein 1-l-Erlenmeyerkolben mit 4 Schikanen, enthaltend 400 ml des Mediums 2, wird mit 8 ml einer Vorkultur von Lasiodiplodia theobromae ATCC 28 570 angeimpft und bei 28° C auf einem Rundschüttler während 24 Stunden (150 rpm) geschüttelt. Nach dieser Zeit zeigt die Kultur ein dichtes Wachstum (100 g/l feuchtes Mycel). Nun werden 0,2 g β-Jonon zur Kultur gegeben und es wird 16 Stunden unter Schütteln fermentiert. Die gaschromatographische Analyse einer mit Methylenchlorid extrahierten Probe (10 ml) zeigt das Verschwinden von β-Jonon und die Bildung einer Vielzahl von Verbindungen daraus. Es werden nun weitere 0,2 g β-Jonon zur Mycel-Kultur zugefügt und es wird weitere 16 Stunden fermentiert. Diese Prozedur wird viermal wiederholt. Hierauf wird der Kolbeninhalt mit 1 n-HCl auf einen pH-Wert von 2 angesäuert und zweimal mit je 200 ml Methylenchlorid extrahiert. Die kombinierten Extrakte werden über $Na_2SO_4$ getrocknet, das $Na_2SO_4$ abfiltriert und das $CH_2Cl_2$ abdestilliert. Es verbleiben 1,1 g eines gelblichen Öls mit einem angenehmen Geruch nach süßem Tabak. Das erhaltene Produkt eignet sich vorzüglich als Riech- und/oder Geschmackstoff wie unter Beispiel 2 beschrieben. Die Riechstoffkomposition wird durch Anwesenheit der Verbindung 5 und 6 charakterisiert.

Beispiel 5

Riechstoffkomposition B, ausgehend von β-Jonon

Ein 70-l-Fermenter, enthaltend 45 l des Mediums 1 und 10 ml Antischaummittel (Polypropylenglykol 2000) wird mit 1,6 l einer 24 Stunden alten Vorkultur von Lasiodiplodia theobromae ATCC 10 936 angeimpft. Die Fermentationsbedingungen sind die folgenden:

Rührsystem: Umwälzrührer (890 rpm), Temperatur: 28° C, Belüftung: 20 l Luft/Minute.

Nach einer Fermentationszeit von 24 Stunden wird ein sehr dichtes Wachstum der Mycel-Kultur erreicht (240 g bei 8000 g abzentrifugiertes feuchtes Mycels pro Liter). Der pH-Wert der Kultursuspension sinkt dabei auf 5,8. Zu diesem Zeitpunkt werden 20 g β-Jonon zur Kultur gegeben, der pH-Wert mittels 1 n-NaOH auf 6,2 eingestellt und mit Hilfe eines automatischen Titrators auf diesem pH-Wert gehalten. Gleichzeitig wird mittels einer Pumpe β-Jonon 4 Tage kontinuierlich (100 g/24 Stunden) zugeführt. Nach beendeter Zugabe wird noch 16 Stunden weiter fermentiert, bis gaschromatographisch nur noch 1—2% β-Jonon nachweisbar sind. Die Aufarbeitung des Fermenterinhaltes geschieht analog zu Beispiel 1. Es werden 402 g eines bräunlichgefärbten Öls mit angenehmem Geruch nach Tabak erhalten, das sich vorzüglich als Riech- und/oder Geschmackstoff eignet, wie unter Beispiel 2 angeführt. Zusätzlich zeigt diese Riechstoffkomposition, in Tabak, insbesondere Zigarettentabak, eingearbeitet, einen erstaunlichen geschmacksverstärkenden Effekt. Die Riechstoffkomposition wird durch Anwesenheit der Verbindung 5 und 6 charakterisiert.

Beispiel 6

Riechstoffkomposition C, ausgehend von α-Iron

Die Herstellung der Riechstoffkomposition erfolgt gemäß Beispiel 2, mit dem Unterschied, daß anstatt β- Jonon α-Iron eingesetzt wird. Es werden insgesamt 28 g α-Iron biotransformiert und man erhält 30,2 g eines hellgelben Öls mit angenehmem blumigem Geruch nach Honig und Tabak. Die Riechstoffkomposition wird durch Anwesenheit der Verbindung 10 charakterisiert.

Beispiel 7

Riechstoffkomposition D, ausgehend von β-Iron

Die Herstellung der Riechstoffkomposition erfolgt gemäß Beispiel 4, mit dem Unterschied, daß anstatt β-Jonon β-Iron (total 1,2 g) zur Mycel-Kultur von Lasiodiplodia theobromae ATCC 10 936 zugegeben wird. Es werden 1,0 g eines hellbräunlichen Öls mit angenehmem Geruch nach Tabak isoliert. Die Riechstoffkomposition wird durch Anwesenheit der Verbindung 11 und 12 charakterisiert.

Beispiel 8

Riechstoffkomposition D, ausgehend von β-Iron

Die Herstellung dieser Riechstoffkomposition erfolgt gemäß Beispiel 1. Es werden insgesamt 25 g β-Iron eingesetzt. Isoliert werden 26,5 g eines hellbräunlichen Öls, das einen angenehmen Geruch nach Tabak aufweist und sich vorzüglich als Riechstoff für Kompositionen dieser Richtung eignet. Die Riechstoffkomposition wird durch Anwesenheit der Verbindung 11 und 12 charakterisiert.

Beispiel 9

Riechstoffkompositionen

A Chypre-Komposition Richtung Herren-Linie mit einem Gehalt an Produkt B

| | Gewichtsteile |
|---|---|
| Methyl-1-methylcyclododecyläther | 200 |
| Bergamotteöl | 140 |
| Fichtennadelöl | 100 |
| Hydroxycitronellal | 100 |
| Citronellol | 80 |
| Petitgrainöl | 60 |
| Musc 174[R] Givaudan (Oxa-4-pentadecanolid) | 60 |
| Galbanumöl | 40 |
| Corianderöl | 40 |
| Cedernholzöl | 40 |
| Patchouliöl | 40 |
| Zitronenöl | 40 |
| Elemiöl | 20 |
| | 940 |

Gibt man zu dieser konventionellen Chypre-Komposition für Herren-Linien 20 Teile des Produktes B, so wird diese betont trocken. Die Holznoten werden sehr angenehm und vorteilhaft unterstrichen.

Gibt man jedoch 40 Teile des Produktes B zu, so wird die Base in Richtung Tabak verändert. Außerdem ist nun im neuen Produkt die in der ursprünglichen Base etwas hervorstechende Elemi-Note vorteilhaft eingekleidet.

B Parfümerie-Base Richtung Chypre mit einem Gehalt an Produkt B

| | Gewichtsteile |
|---|---|
| Ambrette-Moschus | 240 |
| Phenyläthylalkohol | 140 |
| Bergamotteöl | 100 |
| Baummoos absolut | 60 |
| Vetivenylacetat | 60 |
| Jasmin, synthetisch | 60 |
| Linalylacetat | 60 |
| Hydroxycitronellal | 40 |
| Patchouliöl | 30 |
| Eugenol | 30 |
| Methyl-1-methylcyclododecyläther | 30 |
| Styrallylacetat | 30 |
| Sandela[R] Givaudan (3-Isocamphyl-(5)-cyclohexanol) | 30 |
| Neroliöl | 20 |
| Rose, synthetisch | 20 |
| Castoreum, synthetisch | 10 |
| C-11-Aldehyd (10% in Propylenglykol) | 10 |
| Isobutylchinolin (10% in Propylenglykol) | 10 |
| | 980 |

Obige Art Chypre findet insbesondere Anwendung für weibliche Linien. Gibt man 20 Teile des Produktes B zu, so wirkt das Chypre wesentlich schwerer, rosiger, es wird eine vorher nur schwach vorhandene ambrige, honigartige Note verstärkt, welche der ganzen Base eine größere Diffusion verleiht. Die Haftfestigkeit ist nun größer, was sich in der fixativen Eigenschaft des neuen Produktes niederschlägt.

C Parfümerie-Base in Richtung Tee mit einem Gehalt an Produkt B

| | Gewichtsteile |
|---|---|
| Linalylacetat | 300 |
| Linalool | 150 |
| Hydroxycitronellal | 120 |
| Methyl-1-methylcyclododecyläther | 120 |
| Methyldihydrojasmonat | 80 |
| Patchouliblätteröl | 60 |
| Methyleugenol | 40 |
| Musc 174[R] Givaudan | 30 |
| Basilikumöl | 20 |
| Acetanisol | 20 |
| Bornylacetat | 20 |
| Baummoos, absolut | 20 |
| | 980 |

Gibt man zu dieser Parfümerie-Base in Richtung Tee 20 Teile des Produktes B, so wird erstere viel krautiger, kräftiger. Sie erhält eine sehr schön holzig-krautige, trockene und würzige Note, welche sich eindrücklich im 24-h-Wert bemerkbar macht: Die fixierenden Eigenschaften des Produktes B sind eindeutig. Verdünnt man die neue Base mit Alkohol auf 10%, wie sie ihre Anwendung finden würde, kann man die ausgezeichnete Verbindung des Methyldihydrojasmonates mit dem Basilikumöl feststellen. Die erhaltene Base strahlt viel mehr Wärme aus, auch ihr Volumen hat sich stark gesteigert.

D Parfümerie-Base in Richtung blumiger Chypre mit einem Gehalt an Produkt B

| | Gewichtsteile |
|---|---|
| Bergamotteöl | 300 |
| Benzylsalicylat | 100 |
| Hydroxycitronellal | 100 |
| Geraniol | 100 |
| Methyldihydrojasmonat | 90 |
| Keton-Moschus | 80 |
| Benzylacetat | 40 |
| Patchouliöl | 40 |
| Phenyläthylalkohol | 40 |
| Vetivenylacetat | 20 |
| Ambrette Moschus | 20 |
| Styrallylacetat | 10 |
| Galaxolide[R] IFF (1,3,4,6,7,8-Hexahydro-4,6,6,7,8,8-hexamethyl-cyclopenta-$\gamma$-2-benzopyran) | 10 |
| Eugenol | 10 |
| $\gamma$-Undecalacton | 10 |
| Methylnonylacetaldehyd (10% in Propylenglykol) | 10 |
| | 980 |

Gibt man zu diesem Chypre-Typ 20 Teile des neuen Produktes B, so wird dieser viel wärmer in Richtung Trockenfrucht. Diese warmfruchtige Note, welche sich sehr gut in Kompositionen Richtung Tabak-Rose verwerten läßt, kommt ganz besonders in frischer und in auf 15% verdünnter Form zur Geltung.

E Parfümerie-Komposition in Richtung Tabak mit einem Gehalt an Produkt B

| | Gewichtsteile |
|---|---|
| ortho-tert.-Butylcyclohexylacetat | 400 |
| Jasmin, synthetisch | 400 |
| Keton-Moschus | 40 |
| Sandela[R] Givaudan | 40 |
| Styrallylacetat | 30 |
| Coumarin | 20 |
| Isobutylchinolin (10% in Propylenglykol) | 10 |
| Lavendelöl | 10 |
| Vetiveröl | 10 |
| Melilotus, absolut farblos | 10 |
| Galbanumöl | 10 |
| | 980 |

Die obige Tabak-Komposition erhält durch den Zusatz von 20 Teilen des neuen Produktes eine ausgeprägte trockenfruchtartige Note, welche der Komposition mehr Volumen, mehr Vibration und eine generell sehr interessante Note verleihen. Riecht man die Streifen nach 24 Stunden, so erkennt man die fixativen Eigenschaften des neuen Produktes sehr deutlich. Die Coumarin-Note ist unterstrichen, die Tabak-Note wirkt »echt«.

F Parfümerie-Base in Richtung Tee mit einem Gehalt an den Produkten A oder C

| | Gewichtsteile |
|---|---|
| Bergamotteöl | 200 |
| Linalool | 100 |
| Hydroxycitronellal | 100 |
| Methyl-1-methylcyclododecyläther | 100 |
| Methyldihydrojasmonat | 60 |
| Patchouliblätteröl | 60 |
| Geraniol Palmarosa | 60 |
| Galaxolide[R] IFF | 60 |
| Methyleugenol | 40 |
| Bergamylacetat | 40 |
| $\beta$-Jonon | 40 |
| Acetanisol | 40 |
| Allyl-phenoxyacetat | 20 |
| Bornylacetat | 20 |
| Indol (10% in Propylenglykol) | 10 |
| Basilikumöl | 40 |
| | 990 |

Gibt man zu dieser Tee-Base 10 Teile des Produktes A, so wirkt diese viel würziger, der Komplex Patchouli-Basilikum wird sehr angenehm verstärkt und unterstrichen, was sich auch sehr gut im Fond feststellen läßt. Die ausgeprägten fixierenden Eigenschaften von A kommen eindrücklich zur Geltung.

Gibt man jedoch 10 Teile des Produktes C zu der Base, so wirkt sie viel süßer, in Richtung Honig; auch hier ist die Geruchsverstärkung durch den Zusatz an C feststellbar.

G Parfümerie-Base in Richtung Geißblatt mit einem Gehalt an Produkt A bzw. Produkt D

| | Gewichtsteile |
|---|---|
| Methyldihydrojasmonat | 300 |
| Linalool | 100 |
| Hydroxycitronellal | 100 |
| $\alpha$-Hexylzimtaldehyd | 80 |
| Jasmin, synthetisch | 100 |
| Acetanisol | 60 |
| Sandela[R] Givaudan | 40 |
| Phenyläthylalkohol | 40 |
| Benzylacetat | 40 |
| Benzoe resinoid Siam | 30 |
| Linalylanthranilat | 20 |
| Ylang-Ylang-Öl | 20 |
| Indol (10% in Propylenglykol) | 20 |
| Citronellol | 20 |
| $\gamma$-Undecalacton | 10 |
| | 980 |

Gibt man zu dieser blumigen Base 20 Teile des Produktes A, so wirkt erstere viel kräftiger, süßer und erhält eine leichte Honig-Note, welche sich sehr gut mit der allgemeinen Geißblatt-Note verbindet und dieser insbesondere mehr Geruchsvolumen vermittelt. Ein ganz anderer Effekt ist erzielbar, wenn man 20 Teile des Produktes D zufügt. Die Base wirkt nun frischer, leichter und im Fond macht sich eine angenehm pudrige Note bemerkbar, welche in der ursprünglichen Base nicht vorhanden war. Das Geruchsvolumen wird erhöht.

H Blumige Base in Richtung Mimose mit einem Gehalt an Produkt A bzw. Produkt D

| | Gewichtsteile |
|---|---|
| Terpineol | 300 |
| Hydroxycitronellal | 240 |
| Benzylacetat | 60 |
| Keton-Moschus | 60 |
| Heliotropin | 60 |
| Linalool | 40 |
| Methyldihydrojasmonat | 40 |
| Bergamotteöl | 40 |
| $\alpha$-Jonon | 40 |
| Jasmin, synthetisch | 40 |
| Ansialdehyd | 20 |
| Ylang-Ylang-Öl | 20 |
| Eugenol | 20 |
| | 980 |

Durch den Zusatz von 20 Teilen des Produktes A wird in obiger Mimosen-Base die Methyldihydrojasmonat-Note sehr gut mit der Jonon-Note verbunden, es ergibt sich so eine sehr weiche Note. Dieser Effekt wird noch sehr verstärkt, wenn man die Streifen nach 24 Stunden nochmals evaluiert.

Gibt man 20 Teile der Substanz D zu der Mimosen-Base, so wirkt diese viel frischer, sauberer im Geruch, durch das Betonen der Jasmin-Note wird auch der blumige Charakter hervorgehoben.

I Parfümerie-Base in Richtung Heu mit einem Gehalt an Produkt A bzw. C

| | Gewichtsteile |
|---|---|
| Bergamotteöl | 200 |
| Cumarin | 100 |
| Jasmin, synthetisch | 100 |
| Phenyläthylalkohol | 100 |
| Lavendelöl | 100 |
| Hydroxycitronellal | 60 |
| Vetiverylacetat | 40 |
| Sandela[R] Givaudan | 40 |
| Keton-Moschus | 40 |
| Linalool | 40 |
| Baummoos, absolut | 20 |
| Patchouliöl | 20 |
| Methylsalicylat | 20 |
| Ylang-Ylang-Öl | 20 |
| Eugenol | 20 |
| Indol (10% in Propylenglykol) | 10 |
| Zibet, absolut (10% in Propylenglykol) | 10 |
| | 940 |

Gibt man zu dieser Heu-Base 60 Teile des Produktes A, so entsteht aus dieser Base eine harmonisch abgerundete Komposition, die Bergamotte-Note wird hervorgehoben, was mehr Leichtigkeit des Geruchs ergibt. Das Produkt A bewirkt hier insbesondere einen verbindenden Effekt.

Gibt man jedoch 30 Teile C zu dieser Heu-Base, so resultiert im 24-h-Wert ein schöner blumiger Effekt, die etwas zu pudrig erscheinende ursprüngliche Base wirkt viel leichter und frischer. Überraschenderweise wird auch die animalische leicht honigartige Note der Base durch C betont.

K Parfümerie-Base in Richtung Ginster mit einem Gehalt an Produkt A

| | Gewichtsteile |
|---|---|
| Linalool | 200 |
| Bergamotteöl | 200 |
| Petitgrainöl Paraguay | 200 |
| Geranium, synthetisch | 100 |
| Geraniol | 60 |
| $\alpha$-Jonon | 60 |
| Ambrette-Moschus | 40 |
| Moschus-Keton | 40 |
| Methyl-para-cresol | 20 |
| Anisaldehyd | 20 |
| Zitronenöl | 20 |
| Ylang-Ylang-Öl | 20 |
| | 980 |

Gibt man zu dieser Base 20 Teile A, so wird diese sofort viel kräftiger, origineller mit einer angenehm würzigen Note. Die Petitgrain-Note wird leicht unterstrichen.

Beispiel 10

Himbeeraroma unter Verwendung von Produkt B (Beispiel 2)

| Komponenten | Komposition A | B | C |
|---|---|---|---|
| Himbeerketon | 2,5 | 2,5 | 2,5 |
| Essigsäure 80% | 2,0 | 2,0 | 2,0 |
| Dimethylsulfid (1% in Propylenglykol) | 0,15 | 0,15 | 0,15 |
| β-Jonon (1% in Propylenglykol) | – | 1,5 | – |
| Trans-3-Hexenal (50%) | 0,1 | 0,1 | 0,1 |
| Benzylacetat | 0,3 | 0,3 | 0,3 |
| Produkt B (1% in Äthanol) | – | – | 1,5 |

Propylenglykol ad 100 g

Organoleptische Beurteilung

Die Komposition A (konventionelle Himbeer-Base) zeigt einen angenehmen fruchtigen Charakter Richtung »Tutti Frutti«.

Komposition B: Gibt man zur Komposition A β-Jonon, so erhält der fruchtige Charakter dieser Himbeer-Base einen angenehm holzigen Charakter und wirkt nun bereits himbeerähnlich.

Komposition C: Gibt man zur Komposition A jedoch das Produkt B zu, so erhält die Komposition A einen ausgesprochenen Himbeer-Geruch und vor allem Geschmack, der äußerst natürlich wirkt.

Beispiel 11

| Aprikosenaroma | Gewichtsteile A | B |
|---|---|---|
| Angelikawurzelöl | 0,5 | 0,5 |
| Vanillin | 2,0 | 2,0 |
| C 16-Aldehyd | 2,5 | 2,5 |
| Benzaldehyd | 2,5 | 2,5 |
| C 18-Aldehyd | 5,0 | 5,0 |
| Orangenöl | 5,0 | 5,0 |
| Essigsäure-linalylester | 30,0 | 30,0 |
| C 14-Aldehyd | 15,0 | 15,0 |
| Riechstoffkomposition B | – | 20,0 |
| Äthylalkohol | 937,5 | 917,5 |
| | 1000,0 | 1000,0 |

Durch den Zusatz der Riechstoffkomposition B (Beispiel 5) zu obiger Komposition A wird der geruchliche und geschmackliche Eindruck vorteilhaft verändert, indem nun eine ausgeprägte fruchtige Note, Richtung Dörraprikose, in Erscheinung tritt (Dosis für den hergestellten Zuckersirup: 50 g/100 Liter, verdünnt 1 : 5, d. h. 50 g Riechstoffkomposition des Beispiels 5 in 600 Litern Getränk).
Das Aprikosenaroma eignet sich auch vorzüglich zur Aromatisierung von Tabak.

Beispiel 12

Verwendung von Produkt B in Zigarettentabak

Zigaretten wurden mit einer 0,5%igen alkoholischen Lösung des Produktes B (hergestellt nach Beispiel 5) behandelt, und zwar wurden 2 Mikroliter pro Zigarette eingespritzt. Dasselbe wurde mit einer 0,25%igen alkoholischen Lösung des Produktes B wiederholt. Zur organoleptischen Evaluation wurden die behandelten Zigaretten durch ein Panel verraucht und mit unbehandelten Zigaretten verglichen:
Sowohl bei den Zigaretten, die mit einer 0,5%igen alkoholischen Lösung des Produktes B, als auch bei denen, die mit der 0,25%igen Lösung von B behandelt wurden, wurde eine ausgesprochene Verstärkung und Verbesserung des Tabakgeschmackes, verglichen mit den der unbehandelten Zigaretten, festgestellt; zudem wurde das Mundgefühl als hervorragend und weich beschrieben (»salivating effect«).

Die in dieser Beschreibung und den Ansprüchen unten erwähnten Stämme 1)—6) wurden beim ATCC, American Type Culture Collection, Rockville, Maryland, unter den Nummern 7)—12) wieder hinterlegt am 30. Oktober 1979:

| | | | |
|---|---|---|---|
| 1) | Botryodiplodia theobromae | IFO-6469 | 7) ATCC-20 571 |
| 2) | Botryosphaeria rhodina | CBS-356.59 | 8) ATCC-20 572 |
| 3) | Botryosphaeria rhodina | CBS-175.26 | 9) ATCC-20 573 |
| 4) | Botryosphaeria rhodina | CBS-306.58 | 10) ATCC-20 574 |
| 5) | Lasiodiplodia theobromae | ATCC-10 936 | 11) ATCC-20 575 |
| 6) | Lasiodiplodia theobromae | ATCC-28 570 | 12) ATCC-20 576 |

**Patentansprüche**

1. Verfahren zur Herstellung eines Riech- und/oder Geschmackstoffgemisches, dadurch gekennzeichnet, daß man eine Verbindung der Formel

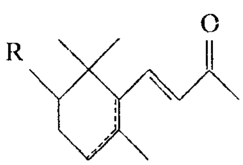

(I)

worin R Wasserstoff oder Methyl und eine der gestrichelten Linien eine zusätzliche Bindung darstellt,

mit einem Fungus des Genus Botryodiplodia, Botryosphaeria oder Lasiodiplodia fermentiert.

2. Verfahren nach Anspruch 1, dadurch gekennzeichnet, daß man als Fungus Botryodiplodia theobromae IFO-6469 (ATCC 20 571), Botryosphaeria rhodina CBS 356.59 (ATCC 20 572), CBS-175.26 (ATCC 20 573) oder CBS-30.658 (ATCC 20 574), Lasiodiplodia theobromae ATCC 28 570 (ATCC 20 576) oder ATCC 10 936 (ATCC 20 575) verwendet.

3. Verfahren nach Anspruch 1 oder 2, dadurch gekennzeichnet, daß man $\alpha$-Jonon als Substrat verwendet.

4. Verfahren nach Anspruch 1 oder 2, dadurch gekennzeichnet, daß man $\beta$-Jonon als Substrat verwendet.

5. Verfahren nach Anspruch 1 oder 2, dadurch gekennzeichnet, daß man $\alpha$-Iron als Substrat verwendet.

6. Verfahren nach Anspruch 1 oder 2, dadurch gekennzeichnet, daß man $\beta$-Iron als Substrat verwendet.

7. Riech- und/oder Geschmackstoffgemisch, erhältlich nach dem Verfahren einer der Ansprüche 1—6.

8. Riech- und/oder Geschmackstoffkomposition, gekennzeichnet durch einen Gehalt an einem Gemisch gemäß Anspruch 7.

9. Verfahren zur Parfümierung von Kompositionen bzw. zur Modifizierung des Geruchs von Riechstoffkompositionen, dadurch gekennzeichnet, daß man diesen Kompositionen eine organoleptisch wirksame Menge eines Riechstoffgemisches gemäß Anspruch 7 oder eine Riechstoffkomposition gemäß Anspruch 8 einverleibt.

10. Verfahren zur Aromatisierung von Nahrungs-, Genußmitteln und Tabak, dadurch gekennzeichnet, daß man Nahrungs-, Genußmitteln oder Tabak ein Geschmackstoffgemisch gemäß Anspruch 7 bzw. eine Geschmackstoffkomposition gemäß Anspruch 8 zugibt.

11. Nahrungs- oder Genußmittel, enthaltend ein Geschmackstoffgemisch gemäß Anspruch 7 bzw. eine Geschmackstoffkomposition gemäß Anspruch 8.

12. Verwendung eines Gemisches gemäß Anspruch 7 oder einer Komposition gemäß Anspruch 8 zu Parfümierungs- bzw. Aromatisierungszwecken.

## Claims

1. Process for the manufacture of a mixture of odorants and/or flavourants, characterized in that one ferments a compound of the general formula

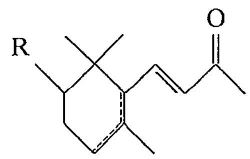

(I)

wherein R represents hydrogen or methyl and one of the dotted lines represents an additional bond,

with a fungus of the genus Botryodiplodia, Botryosphaeria or Lasiodiplodia.

2. A process according to claim 1, wherein Botrysphaeria theobromae IFO-6469 (ATCC 20 571), Botryosphaeria rhodina CBS 356.59 (ATCC 20 572), CBS-175.26 (ATCC 20 573) or CBS-30.658 (ATCC 20 574) or Lasiodiplodia theobromae ATCC 28 570 (ATCC 20 576) or ATCC 10 936 (ATCC 20 575) is used as the fungus.

3. A process according to claim 1 or claim 2, wherein $\alpha$-ionone is used as the substrate.

4. A process according to claim 1 or claim 2, wherein $\beta$-ionone is used as the substrate.

5. A process according to claim 1 or claim 2, wherein $\alpha$-irone is used as the substrate.

6. A process according to claim 1 or claim 2, wherein $\beta$-irone is used as the substrate.

7. A mixture of odorants and/or flavourants, obtainable according to the process claimed in any one of claims 1 to 6.

8. An odorant and/or flavouring composition, characterized by a content of a mixture in accordance with claim 7.

9. A process for the perfuming of compositions or for the modification of the odour of odorant compositions, which process comprises incorporating into said compositions an organoleptically effective amount of a mixture of odorants in accordance with claim 7 or of an odorant composition in accordance with claim 8.

10. A process for the flavouring of foodstuffs, luxury consumables and tobacco, characterized in that one adds a mixture of flavourants in accordance with claim 7 or a flavouring composition in accordance with claim 8 to foodstuffs, luxury consumables or tobacco.

11. A foodstuff of luxury consumable, characterized by a content of a mixture of flavourants in accordance with claim 7, or a flavouring composition in accordance with claim 8.

12. The use of a mixture in accordance with claim 7 or of a composition in accordance with claim 8 for perfuming or flavouring purposes.

## Revendications

1. Procédé de préparation d'un mélange odorant et/ou aromatisant, caractérisé en ce qu'on fait fermenter un composé de formule:

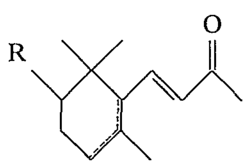

(I)

où R représente un hydrogène ou un méthyle et où l'une des lignes pointillées représente une liaison supplémentaire, avec un champignon du genre Botryodiplodia, Botryosphaeria ou Lasiodiplodia.

2. Procédé selon la revendication 1, caractérisé en ce qu'on utilise comme champignon Botryodiplodia Theobromae IFO-6469 (ATCC 20 571), Botryosphaeria rhodina CBS-356.59 (ATCC 20 572), CBS-175.26 (ATCC 20 573) ou CBS-30.658 (ATCC 20 574), Lasiodiplodia theobromae ATCC 28 570 (ATCC 20 576) ou ATCC 10 936 (ATCC 20 575).

3. Procédé selon les revendications 1 ou 2, caractérisé en ce qu'on utilise de l'alpha-ionone comme substrat.

4. Procédé selon les revendications 1 ou 2, caractérisé en ce qu'on utilisie de la bêta-ionone comme substrat.

5. Procédé selon les revendications 1 ou 2, caractérisé en ce qu'on utilise de l'alpha-irone comme substrat.

6. Procédé selon les revendications 1 ou 2, caractérisé en ce qu'on utilisie de la bêta-irone comme substrat.

7. Mélange odorant et/ou aromatisant pouvant être obtenu selon le procédé d'une des revendications 1—6.

8. Composition odorante et/ou aromatisante, caractérisé en ce qu'elle contient un mélange selon la revendication 7.

9. Procédé pour parfumer des compositions ou pour modifier l'odeur de compositions odorantes, caractérisé en ce qu'on incorpore à ces compositions une quantité organoleptiquement efficace d'un mélange odorant selon la revendication 7 ou une composition odorante selon la revendication 8.

10. Procédé d'aromatisation des aliments, des agents de consommation de luxe et du tabac, caractérisé en ce qu'on ajoute à des aliments, des agents de consommation de luxe ou du tabac un mélange odorant selon la revendication 7 ou une composition odorante selon la revendication 8.

11. Aliment ou agent de consommation de luxe contenant un mélange d'agent aromatisant selon la revendication 7 ou une composition aromatisante selon la revendication 8.

12. Application d'un mélange selon la revendication 7 ou d'une composition selon la revendication 8 à des fins de parfumerie ou d'aromatisation.